Europäisches Patentamt

European Patent Office

Office européen des brevets

(11) Veröffentlichungsnummer : **0 401 653 B1**

(12) # EUROPÄISCHE PATENTSCHRIFT

(45) Veröffentlichungstag der Patentschrift :
**13.01.93 Patentblatt 93/02**

(51) Int. Cl.⁵ : **A61K 31/495**

(21) Anmeldenummer : **90110207.9**

(22) Anmeldetag : **30.05.90**

(54) **Verwendung von Naftopidil zur Therapie der Dysurie bei benigner Prostata-Hyperthrophie.**

Die Akte enthält technische Angaben, die nach dem Eingang der Anmeldung eingereicht wurden und die nicht in dieser Patentschrift enthalten sind.

(30) Priorität : **07.06.89 DE 3918543**

(43) Veröffentlichungstag der Anmeldung :
**12.12.90 Patentblatt 90/50**

(45) Bekanntmachung des Hinweises auf die
Patenterteilung :
**13.01.93 Patentblatt 93/02**

(84) Benannte Vertragsstaaten :
**AT BE CH DE DK ES FR GB GR IT LI LU NL SE**

(56) Entgegenhaltungen :
**DRUGS OF THE FUTURE, Band 12, Nr. 1, Januar 1987, Seiten 31-33; "Naftopidil"**

(56) Entgegenhaltungen :
**UROLOGY, Band 32, Dezember 1988, Seiten 27-31; U. DUNZENDORFER: "Clinical experience: symptomatic management of BPH withterazosin"**
**EUROPEAN JOURNAL OF PHARMACOLOGY, Band 107, Nr. 2, Januar 1985, Seiten 111-117; J.P. HIEBLE et al.: "In vitrocharacterization of the alpha-adrenoceptors in human prostate"**
**BRITISH JOURNAL OF UROLOGY, Band 48, Nr. 4, 19. August 1976, Seiten 255-263; M. CAINE et al.: "The use of alpha-adrenergic-blockers in benign prostatic obstruction"**

(73) Patentinhaber : **BOEHRINGER MANNHEIM GMBH
Sandhofer Strasse 116
W-6800 Mannheim 31 (DE)**

(72) Erfinder : **Sponer, Gisbert, Dr. med. vet.
Lessingstrasse 13
W-6941 Laudenbach (DE)**
Erfinder : **Reicke, Ulrich
Ladenburgerstrasse 31a
W-6945 Hirschberg-Leutershausen (DE)**
Erfinder : **Nelböck-Hochstetter, Michael,
Dr.-phil.
Bareiselweg 33
W-8132 Tutzing (DE)**

## Beschreibung

Gegenstand der Erfindung ist die Verwendung von Naftopidil zur Therapie der Dysurie bei benigner Prostata-Hypertrophie.

Naftopidil, chemische Bezeichnung 1-(2-Methoxyphenyl)-4-[3-(naphth-1-yl-oxy)-2-hydroxy-propyl]-piperazin, ist Gegenstand des Patentes DE-B-24 08 804 und wird dort als eine Verbindung mit ausgeprägten blutdrucksenkenden und damit antihypertensiven Eigenschaften beschrieben. Ferner soll es die durch Dextran ausgelösten anaphylaktoiden Reaktionen bei Ratten hemmen.

In "Drugs of the Future" Bd. 12, Nr. 1, 1987 ist Naftopidil als ein selektiver α-Blocker beschrieben.

In "Britisch Journal of Urology" (1976), Bd. 48, Nr. 4, Seite 255-263 ist aufgezeigt, daß "alpha-adrenergic blockers" bei der Behandlung von benignen Prostataobstruktionen eingesetzt werden können.

In "Urology", Bd. 32, Seite 27-31 wird beschrieben, daß Terazosin zur Behandlung der benignen Prostata-Hypertrophie Anwendung finden kann.

Bei klinischen Untersuchungen von Naftopidil wurde nun gefunden, daß die Verbindung Beschwerden bei Prostata-Hypertrophie lindert. Die Beschwerden sind gekennzeichnet durch Störung beim Harnlassen, insbesondere Harndrang während der Nacht. Betroffen sind hiervon in der Regel Männer über 60 Jahre. Eine spezifische Therapie ist bisher nicht etabliert. Versuchsweise werden Wirkstoffe auf Basis von pflanzlichen Extrakten oder β-Sitosterin eingesetzt (vgl. Rote Liste 1989).

Die Wirksamkeit von Naftopidil für die Behandlung der Dysurie auf der Grundlage einer Prostata-Hypertrophie wird in der nachstehend näher beschriebenen klinischen Studie belegt.

Die Untersuchungen wurden an insgesamt 39 Patienten durchgeführt. 37 von ihnen litten an einer Prosta-Hypertrophie. Nach einer einwöchigen Vorausperiode, in der Placebomedikation erfolgte, wurde Naftopidil in Dosen zwischen 12,5 und 100 mg täglich verabfolgt. Die Dosen richteten sich nach der Effektivität, bei nicht ausreichender Wirkung wurde die Dosis im Abstand von einer Woche, beginnend mit 12,5 mg, um das Doppelte erhöht.

Als Kriterien wurden unter anderem gewählt:

Häufigkeit für das Urinieren während des Tages und der Nacht, Dauer des Urinlassens, Urinfluß, Abdominaldruck, um Urin lassen zu können sowie Empfinden beim Urinlassen.

Die Bewertung erfolgte aufgrund einer Skala in folgender Klassifikation:

Deutlich gebessert, gebessert, leicht gebessert, unverändert, verschlimmert.

34 Patienten konnten insgesamt in die Auswertung genommen werden. Von diesen wurden 8 deutlich gebessert, 14 gebessert, 7 leicht gebessert, 1 Patient hatte ein unverändertes Verhalten, bei 4 weiteren Patienten konnte keine eindeutige Entscheidung getroffen werden.

Naftopidil wird vorzugsweise oral appliziert.

Die Herstellung von Naftopidil erfolgt nach den in der DE-B-24 08 804 beschriebenen Verfahren.

Zur Herstellung von Salzen setzt man die erfindungsgemäße Verbindung mit pharmakologisch verträglichen organischen oder anorganischen Säuren, z.B. Salzsäure, Schwefelsäure, Phosphorsäure, Milchsäure, Zitronensäure oder Alkylsulfonsäure, um.

Zur Herstellung von Arzneimitteln wird Naftopidil in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechen Hilfsstoffe in Wasser oder Öl, wie z.B. Olivenöl, suspendiert oder gelöst.

Naftopidil wird üblicherweise in Mengen zwischen 10 mg bis 100 mg pro Tag appliziert. Bevorzugt ist eine Ein- bis Zweimal gabe pro Tag in einer Einzeldosis von 10-50 mg.

Eine beispielhafte Rezeptur für eine Tablette mit 25,0 mg Wirkstoff setzt sich wie folgt zusammen:

```
Naftopidil                              25,0 mg
Lactose x 1 H₂O                        110,0 mg
Poly-(1-Vinyl-2-pyrrolidon)
    MG   25000                           3,0 mg
mikrokristalline Cellulose              15,0 mg
hochdisperses SiO₂                       1,5 mg
Poly-(0-carboxymethyl)-
    Stärke, Na-Salz                      4,0 mg
Magnesium-Stearat                        1,5 mg
                                       ─────────
                                       160.0 mg
```

**Patentansprüche**

1. Verwendung von 1-(2-Methoxyphenyl)-4-[3-(naphth-1-yl-oxy)-2-hydroxy-propyl]-piperazin oder dessen pharmakologisch unbedenliche Salze zur Herstellung von Arzneimitteln zur Behandlung der Dysurie bei Prostata-Hypertrophie.

2. Oral applizierbares Zusammensetzung enthaltend als Wirkstoff 1-(2-Methoxyphenyl)-4-[3-(naphth-1-yl-oxy)-2-hydroxy-propyl]-piperazin, dadurch gekennzeichnet, daß es in der Einheitsdosis den Wirkstoff in einer Menge von 10-50 mg neben üblichen Träger- und Hilfsstoffen enthält.

**Claims**

1. Use of 1-(2-methoxyphenyl)-4-[3-(naphth-1-yloxy)-2-hydroxypropyl]-piperazine or of its pharmacologically acceptable salts for the preparation of medicaments for the treatment of dysuria in the case of prostatic hypertrophy.

2. Orally administerable composition containing as active material 1-(2-methoxyphenyl)-4-[3-(naphth-1-yloxy)-2-hydroxypropyl]-piperazine, characterised in that, in a single dose, it contains the active material in an amount of 10 - 50 mg, in addition to usual carrier and adjuvant materials.

**Revendications**

1. Utilisation de la 1-(2-méthoxyphényl)-4-[3-(naphtyl-1-yl-oxy)-2-hydroxy-propyl]-pipérazine, ou de ses sels pharmaceutiquement acceptables, pour la préparation de médicaments destinés au traitement de la dysurie dans l'hypertrophie de la prostate.

2. Composition destinée à l'administration par voie orale, contenant, comme substance active, la 1-(2-méthoxyphényl)-4-[3-(naphtyl-1-yl-oxy)-2-hydroxy-propyl]-pipérazine, caractérisée en ce que la prise unitaire contient la substance active en une quantité de 10-50 mg, avec les supports et adjuvants usuels.